# EUROPEAN PATENT SPECIFICATION

(11) **EP 1 773 279 B1**
(45) Date of publication and mention of the grant of the patent: **02.09.2015**
(21) Application number: 05770086.6
(22) Date of filing: 05.07.2005
(51) Int. Cl.: A61K 8/27, A61Q 11/00, A61K 8/19

(54) **TOOTHPASTE COMPRISING CALCIUM CARBONATE AND ZINC CITRATE**
ZAHNPASTE CALCIUMCARBONAT UND ZINKCITRAT ENTHALTEND
DENTIFRICE COMPRENANT DU CARBONATE DE CALCIUM ET DU CITRATE DE ZINC

(30) Priority: 03.08.2004 EP 04254661
(43) Date of publication of application: 18.04.2007
(73) Proprietor: Unilever N.V., 3013 AL Rotterdam (NL); Unilever PLC, London EC4Y 0DY (GB)
(72) Inventor: Waterfield, Philip C., Bebington, Wirral Merseyside CH63 3JW (GB)
(74) Representative: Tansley, Sally Elizabeth
(86) International application number: PCT/EP2005/007319
(87) International publication number: WO 2006/012967

(56) References cited:
- EP-A- 1 072 253
- WO-A-00/38644
- WO-A-96/09034
- DE-A- 3 021 150
- US-A- 4 289 755
- US-A- 4 325 939
- US-A- 5 188 820
- DATABASE CA [Online] CHEMICAL ABSTRACTS SERVICE, COLUMBUS, OHIO, US; ATANASOV, B. ET AL: "Influence of various abrasive types of the in vitro acid stability of teeth enamel" XP002312639 retrieved from STN Database accession no. 1988:498563 & FARMATSIYA (SOFIA, BULGARIA) , 38(1), 41-4 CODEN: FMTYA2; ISSN: 0428-0296, 1988,

## Description

The present invention relates to an oral care composition comprising bioactive zinc salts in a chalk formulation. The invention also relates to a method for making said composition.

EP-A1-0 740 932 (Unilever) discloses a visually-clear gel type dentifrice comprising a zinc salt which is more water soluble than zinc citrate, an amino acid which can bind zinc and a low refractive index type abrasive silica.

US 5 470 561 (Klugkist) discloses an anti-plaque mouthwash comprising a zinc salt and triclosan. The composition may also comprise glycine and has a pH of between 4 and 8, preferably between 5 and 7, the preferred pH being 6.

GBA-2 052 978 (Unilever) discloses a toothpaste comprising zinc salts with glycine and a pH of from 4.5 to 8.0.

US 5 632 972 (Williams) discloses a method for minimising damage to gingival and periodontal tissue by delivering a first component comprising zinc and a second component comprising a bicarbonate.

WO 96/09034 (Unilever) discloses oral care products with improved sensorially-perceivable cleaning benefit achieved by the inclusion of agglomerates of particulate materials, substantially free from organic and/or inorganic binding agents. The agglomerates may further include materials having a therapeutic benefit on the teeth or gums. An example of such a therapeutic agent is zinc citrate.

US 4 325 939 (Shah) discloses an alkali metal or ammonium zinc citrate prepared for use in dental compositions and especially in mouthwash compositions.

WO 00/38644 (Church and Dwight Co. Inc )discloses storage stable aqueous or aqueous/alcoholic solution of zinc ions in the presence of bicarbonate ions.

EP 1 072 253 (Sunstar KK) discloses an oral composition comprising palatinit which exerts a synergistic effect when combined with a fluorine or zinc compound.

US 5 188 820 (Cummins et al) discloses oral compositions such as dentifrices comprising a mixture of a stannous salt such as stannous fluoride or stannous pyrophosphate and a zinc salt such as zinc citrate.

In a first aspect of the present invention there is provided a toothpaste according to claim 1.

In comprising water-solubilised zinc citrate the toothpaste does not form zinc hydroxide upon storage in a closed container such as a toothpaste tube and hence no gassing on the resulting formation of carbon dioxide.

The molar ratio between the zinc ions and the citrate ligand is from 1:1.3 to 1:1.7.

Preferably, zinc citrate is present at from 0.01 to 5% by weight of the toothpaste composition, preferably from 0.5 to 3.0% by weight of the composition. This ratio between the zinc and the citrate salt provides, in this type of formulation, an optimal balance between making enough zinc ions bioavailable, and capable of interacting with bacteria, without forming a deleterious amount of water-insoluble zinc hydroxide.

Preferably, the excess citrate is incorporated into the composition as an alkali metal citric acid salt such as potassium citrate or sodium citrate.

The toothpaste composition also comprises water. Preferably, it comprises from 5 to 50% by weight and most preferably from 15 to 35% by weight water.

The abrasive system employed in the present invention is calcium carbonate based. This does not prevent the use of non-calcium carbonate abrasives in addition, such as silicas, tungsten carbide and silicon carbide.

The abrasive system is present at from 10 to 70% by weight of the composition comprising 45 to 60% by weight calcium carbonate.

Preferred calcium carbonates include fine ground natural chalk since it has a surprising stability with regard to its interactivity with zinc salts. The term fine ground natural chalk (FGNC) is a known term in the art and suitable examples of such are disclosed in US 2003/0072721 A1 (Riley) the contents of which with regards to the definition, types and grades of FGNC are incorporated herein by reference. Nevertheless, by FGNC is meant chalk which is obtained by milling limestone or marble deposits. Preferably, the FGNC comprises particulate matter of weight-based median particle size ranging from 1 to 15 µm and BET surface area ranging from 0.5 to 3 m²/g.

The toothpaste composition according to the invention also preferably comprises a fluoride ion source such as an alkali metal salt of monofluorophosphate, preferably sodium monofluorophosphate. Such fluoride ion source will be present at such an amount to provide free fluoride ion at from 100 to 2000 ppm, preferably from 900 to 1500 ppm.

Preferably, the toothpaste according to the invention comprises an agent selected from the group consisting of anti-caries agents, anti-tartar agents, anti-malodour agents, whitening teeth agents, anti-gingivitis agents and mixtures thereof.

The toothpaste according to the invention comprise further ingredients which are common in the art, such as:
antimicrobial agents, e.g. chlorhexidine, sanguinarine extract, metronidazole, quaternary ammonium compounds, such as cetylpyridinium chloride; bis-guanides, such as chlorhexidine digluconate, hexetidine, octenidine, alexidine; and halogenated bisphenolic compounds, such as 2,2' methylenebis-(4-chloro-6-bromophenol);
anti-inflammatory agents such as ibuprofen, flurbiprofen, aspirin, indomethacin etc.;
anti-caries agents such as sodium- and stannous fluoride, aminefluorides, sodium monofluorophosphate, sodium trimeta phosphate and casein;
plaque buffers such as urea, calcium lactate, calcium glycerophosphate and strontium polyacrylates;
vitamins such as Vitamins A, C and E;
plant extracts;
desensitising agents, e.g. potassium citrate, potassium chloride, potassium tartrate, potassium bicarbonate, potassium oxalate, potassium nitrate and strontium salts;
anti-calculus agents, e.g. alkali-metal pyrophosphates, hypophosphite-containing polymers, organic phosphonates and phosphocitrates etc.;
biomolecules, e.g. bacteriocins, antibodies, enzymes, etc.;
flavours, e.g. peppermint and spearmint oils;
proteinaceous materials such as collagen;
preservatives;
opacifying agents;
colouring agents;
pH-adjusting agents;
sweetening agents;
pharmaceutically acceptable carriers, e.g. starch, sucrose, water or water/alcohol systems etc.;
surfactants, such as anionic, nonionic, cationic and zwitterionic or amphoteric surfactants;
particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight of the oral care composition. Preferred abrasives are chalk and silica, more preferably fine ground natural chalk.
   Humectants such as glycerol, sorbitol, propyleneglycol, xylitol, lactitol etc.;
binders and thickeners such as sodium carboxymethylcellulose, hydroxyethyl cellulose (Natrosol®), xanthan gum, gum arabic etc. as well as synthetic polymers such as polyacrylates and carboxyvinyl polymers such as Carbopol®;
polymeric compounds which can enhance the delivery of active ingredients such as antimicrobial agents can also be included;
buffers and salts to buffer the pH and ionic strength of the oral care composition; and
other optional ingredients that may be included are e.g. bleaching agents such as peroxy compounds e.g. potassium peroxydiphosphate, effervescing systems such as sodium bicarbonate/citric acid systems, colour change systems, and so on.

Liposomes may also be used to improve delivery or stability of active ingredients.

In a second aspect the invention presents a method of making an oral composition according to the first aspect the method comprising the steps:
- solubilising the zinc citrate in water;
- add a buffering agent until the pH reaches at least 8.5 or the natural pH of the chalk base if higher;
- add the remaining oral care ingredients, including the calcium carbonate abrasive.

Preferably, the solubilising step comprises mixing water, zinc citrate trihydrate and alakali-metal citrate salt and mixing until the zinc citrate is fully solubilised, i.e. the composition is clear.

Preferably, the buffering agent is sodium hydroxide.

Preferably, humectants and preservatives are added after the mixture has been buffered to 8.5 or higher if needed. Suitable humectants include sorbitol.

Preferably, the chalk is added after the humectants and is mixed until the formulation is homogenous. More preferably, the calcium carbonate is added together with the foaming agent which is preferably sodium lauryl sulphate. More preferably, these materials are added stepwise to ensure proper mixing.

Preferably, the thickeners are added after the calcium carbonate and are mixed until homogenous and air-free.

Preferably, a fluoride ion source, such as sodium monofluorophosphate is added before flavours are also added.

Except in the operating and comparative examples, or where otherwise explicitly indicated, all numbers in this description indicating amounts of material ought to be understood as modified by the word 'about'.

The term 'comprising' is meant not to be limiting to any subsequently stated elements but rather to encompass non-specified elements of major or minor functional importance. In other words the listed steps, elements or options need not be exhaustive. Whenever the words 'including' or 'having' are used, these terms are meant to be equivalent to 'comprising' as defined above.

Embodiments according to the invention shall now be discussed with reference to the following non-limiting examples.

### EXAMPLE 1

The following example formulation is an embodiment according to the invention. It is made according to the following steps:
1. Place water, sweetener, zinc citrate trihydrate and tripotassium citrate into the mixer, stir until the zinc citrate is fully solubilised.
2. Add sodium hydroxide until the pH of the mix is 8.5 or above if the natural pH of the chalk suspension is higher.
3. Add sorbitol and formalin and stir until fully dissolved.
4. Add the fine ground natural chalk and sodium lauryl sulphate as a dry mixture and mix until homogenous.
5. Add the thickening silica and sodium carboxymethyl cellulose until homogenous and air-free.
6. Add sodium monofluorophosphate and stir until fully dissolved.
7. Add flavour and stir to complete the process.

| **Ingredient** | **% (w/w)** |
|---|---|
| | |
| Fine Ground Natural Chalk (Addon 1015) | 40.00 |
| Sorbitol (70% aq) | 15.00 |
| Thickening silica | 3.00 |
| Sodium carboxymethyl cellulose | 0.90 |
| Flavour | 1.10 |
| Sweetener | 0.23 |
| Sodium lauryl sulphate | 2.50 |
| Zinc citrate | 2.00 |
| Tripotassium citrate | 3.00 |
| Sodium Hydroxide | 0.385 |
| Formalin | 0.10 |
| Sodium monofluorophosphate | 0.76 |
| Water | to 100 |

### EXAMPLE 2

Table shows the required amount of tripotassium citrate monohydrate required to fully solubilise the zinc citrate trihydrate (ZCT).

Two grammes (3.18x10⁻³ mole) of ZCT was placed into 100g of de-ionised water and stirred to suspend the ZCT.

Independently, 20.63g (6.36x10⁻² mole) of tripotassium citrate monohydrate was dissolved in 100g of de-ionised water (to form a 0.636 Molar solution) and then placed in a burette.

The solution of tripotassium citrate was then titrated into the ZCT suspension until the point when the solution just clarifies. At this point, no residual sparingly soluble ZCT remains in suspension.

**Table**

| Quantity of tripotassium citrate added (ml) | Solution pH | Molar ratio of zinc to total citrate | Observation |
|---|---|---|---|
| 0 | 5.17 | 3:2 | Cloudy solution |
| 2 | 5.83 | | Cloudy solution |
| 4 | 5.98 | | Cloudy solution |
| 5 | | 1:1 | Cloudy solution |
| 6 | 6.04 | | Greyish cloudy solution |
| 8 | 6.04 | | Greyish cloudy solution |
| 10 | 6.07 | | Light greyish cloudy solution |
| 11 | 6.11 | | Almost clear solution, hazy |
| 12 | 6.13 | 2:3 | Clear solution |
| 14 | 6.23 | | Clear |

The quantity of tripotassium citrate that is required to completely clear a 2% solution of ZCT was 12ml of 0.636 Molar solution, equivalent to 7.63x10⁻³ moles of tripotassium citrate. Therefore 2g of ZCT reacts completely with 2.5g of potassium citrate monohydrate giving, in solution, a total zinc:citrate ratio of 1:1.46 (rounded to 2:3)

The original ratio of zinc:citrate in ZCT prior to stepwise addition of further citrate was 3:2. At a zinc:citrate ratio of 1:1 some of the zinc citrate remains unreacted leaving a cloudy solution, however at a zinc:citrate ratio of 2:3 all of the ZCT is reacted and is fully solubilised, i.e. there is no suspended zinc citrate left for the formation of zinc hydroxide and thus carbon dioxide in the tube.

## Claims

1. Toothpaste comprising from 10 to 70% by weight of the composition of a calcium carbonate based abrasive system comprising from 45 to 60% by weight calcium carbonate, water and, water-solubilised zinc citrate, wherein the molar ratio between the zinc ions and citrate ligand is from 1:1.3 to 1:1.7.

2. Toothpaste composition according to any preceding claim, comprising an alkali metal salt of citric acid.

3. Toothpaste composition according to claim 2, wherein the citrate salt is potassium citrate.

4. Toothpaste composition according to claim 2, wherein the citrate salt is sodium citrate.

5. Toothpaste composition according to any preceding claim, wherein the calcium carbonate is fine ground natural chalk.

6. Toothpaste composition according to any preceding claim, wherein the composition comprises a fluoride ion source.

7. Toothpaste composition according to any preceding claim, wherein the zinc citrate is present at from 0.01 to 5% by weight of the composition.

8. Toothpaste according to any preceding claim comprising an agent selected from the group consisting of anti-caries agents, anti-tartar agents, anti-malodour agents, whitening teeth agents, anti-gingivitis agents and mixtures thereof.

9. A method of making a toothpaste, according to Claim 1, the method comprising:
- solubilising the zinc citrate in water;
- adding a buffering agent until the pH reaches at least 8.5 or the natural pH of the calcium carbonate base if higher;
- adding the calcium carbonate; and
- optionally adding any further ingredients at any stage.

## Patentansprüche

1. Zahnpasta,
die 10 bis 70 %, auf das Gewicht der Zusammensetzung bezogen, eines auf Calciumcarbonat basierenden Schleifmittelsystems, das 45 bis 60 Gew.-% Calciumcarbonat aufweist, Wasser und ein in Wasser löslich gemachtes Zinkcitrat aufweist, wobei das Molverhältnis zwischen den Zinkionen und dem Citratliganden 1:1,3 bis 1:1,7 beträgt.

2. Zahnpastazusammensetzung nach einem der vorstehenden Ansprüche,
die ein Alkalimetallsalz von Citronensäure aufweist.

3. Zahnpastazusammensetzung nach Anspruch 2,
wobei das Citratsalz Kaliumcitrat ist.

4. Zahnpastazusammensetzung nach Anspruch 2,
wobei das Citratsalz Natriumcitrat ist.

5. Zahnpastazusammensetzung nach einem der vorstehenden Ansprüche,
wobei das Calciumcarbonat fein gemahlener natürlicher Kalk ist.

6. Zahnpastazusammensetzung nach einem der vorstehenden Ansprüche,
wobei die Zusammensetzung eine Fluoridionenquelle aufweist.

7. Zahnpastazusammensetzung nach einem der vorstehenden Ansprüche,
wobei das Zinkcitrat mit 0,01 bis 5 Gew.-% der Zusammensetzung vorliegt.

8. Zahnpasta nach einem der vorstehenden Ansprüche,
die ein Mittel aufweist, das aus der Gruppe ausgewählt ist, die aus Mitteln gegen Karies, Mitteln gegen Zahnstein, Mitteln gegen Mundgeruch, Zähne aufhellenden Mitteln, Mitteln gegen Zahnfleischentzündungen und Gemischen davon besteht.

9. Verfahren zum Herstellen einer Zahnpasta nach Anspruch 1,
wobei das Verfahren Folgendes aufweist:
- Löslichmachen des Zinkcitrats in Wasser;
- Zugeben eines Puffers, bis der pH-Wert zumindest 8,5 oder den natürlichen pH-Wert der Calciumcarbonatbase, falls dieser höher ist, erreicht;
- Zugeben des Calciumcarbonats; und
- gegebenenfalls Zugeben irgendwelcher weiterer Bestandteile in irgendeiner Stufe.

## Revendications

1. Pâte dentifrice comprenant de 10 à 70 % en poids de la composition d'un système abrasif à base de carbonate de calcium comprenant de 45 à 60 % en poids de carbonate de calcium, d'eau et du citrate de zinc solubilisé dans l'eau, dans laquelle le rapport molaire entre les ions de zinc et le ligand citrate est de 1:1,3 à 1:1,7,

2. Composition de pâte dentifrice selon l'une quelconque des revendications précédentes, comprenant un sel de métal alcalin d'acide citrique.

3. Composition de pâte dentifrice selon la revendication 2 dans laquelle le sel de citrate est le citrate de potassium.

4. Composition de pâte dentifrice selon la revendication 2, dans laquelle le sel de citrate est le citrate de sodium.

5. Composition de pâte dentifrice selon l'une quelconque des revendications précédentes, dans laquelle le carbonate de calcium est de la craie naturelle broyée fine.

6. Composition de pâte dentifrice selon l'une quelconque des revendications précédentes, dans laquelle la composition comprend une source d'ion fluorure.

7. Composition de pâte dentifrice selon l'une quelconque des revendications précédentes, dans laquelle le citrate de zinc est présent de 0,01 à 5 % en poids de la composition.

8. Pâte dentifrice selon l'une quelconque des revendications précédentes comprenant un agent choisi dans le groupe constitué d'agents anti-caries, agents anti-tartre, agents contre les mauvaises odeurs, agents de blanchiment des dents, agents anti-gingivite et des mélanges de ceux-ci.

9. Procédé de fabrication d'une pâte dentifrice selon la revendication 1, le procédé comprenant :
- la solubilisation du citrate de zinc dans de l'eau ;
- l'ajout d'un agent tampon jusqu'à ce que le pH atteigne au moins 8,5 ou le pH naturel du carbonate de calcium basique s'il est plus élevé ;
- l'ajout du carbonate de calcium ; et
- facultativement, l'ajout de composants supplémentaires éventuels à un stade quelconque.
